# EUROPÄISCHE PATENTANMELDUNG

(11) **EP 1 362 605 A1**
(43) Veröffentlichungstag der Anmeldung: **19.11.2003**
(21) Anmeldenummer: 02010185.3
(22) Anmeldetag: 14.05.2002
(51) Int. Cl.: A61M 1/34, A61M 1/16

(54) **Dialysevorrichtung zur Entfernung proteingebundener Substanzen**

(71) Anmelder: Kreymann, Bernhard, Dr., 80798 München (DE)
(72) Erfinder: Kreymann, Bernhard, Dr., 80798 München (DE)
(74) Vertreter: von Hellfeld, Axel, Dr. Dipl.-Phys.

(57) **Zusammenfassung**

Die vorliegende Erfindung betrifft eine Dialysevorrichtung zur Entfernung proteingebundener Substanzen aus einer biologischen Flüssigkeit, insbesondere Blut oder Blutplasma, die wenigstens ein Mittel zum Inlösungbringen von zu entfernenden proteinbindenden Substanzen in die biologische Flüssigkeit und/oder die Dialysierflüssigkeit enthält, sowie ein Verfahren zur Entfernung proteingebundener Substanzen aus einer biologischen Flüssigkeit.

## Beschreibung

Die vorliegende Erfindung betrifft eine Dialysevorrichtung zur Entfernung proteingebundener Substanzen aus einer biologischen Flüssigkeit, insbesondere Blut oder Blutplasma, die wenigstens ein Mittel zum Inlösungbringen von zu entfernenden proteinbindenden Substanzen in die biologische Flüssigkeit und/oder die Dialysierflüssigkeit enthält, sowie ein Verfahren zur Entfernung proteingebundener Substanzen aus einer biologischen Flüssigkeit.

Bei einer Reihe von Erkrankungen, zu denen insbesondere das akute oder chronische Nierenversagen, das akute oder chronische Leberversagen oder exogene Intoxikationen gehören, müssen pathogene Substanzen, die im Plasmawasser gelöst sind oder an Proteine gebunden sind, aus dem Blut entfernt werden. Mittels der konventionellen Verfahren der Hämodialyse, Hämofiltration oder Hämodiafiltration können proteingebundene Substanzen nur in geringem Ausmaß eliminiert werden.

Die fehlende Eliminierung proteingebundener Substanzen spielt für den Ersatz der Nierenfunktion derzeit nur eine untergeordnete Rolle. Die Lebenserwartung von Patienten mit akutem oder chronischem Nierenversagen kann durch einen Nierenersatztherapie normalisiert (bei Erholung der Organfunktion bei akutem Nierenversagen) oder zumindest für Jahre verlängert werden (bei Patienten, die eine Nierenersatztherapie weiter benötigen).

Ganz anders sieht dies bei akutem Leberversagen oder einer akuten Verschlechterung eines chronischen Leberversagens aus. Hier stehen derzeit mit der Nierenersatztherapie vergleichbare Methoden nicht zur Verfügung. Die Leberfunktion kann im wesentlichen in zwei Hauptfunktionen unterteilt werden:
- die Synthese lebenswichtiger Proteine und
- die Entgiftung von hauptsächlich proteingebundenen Toxinen.

Für einen Ersatz der Synthesefunktion steht derzeit im Prinzip nur die Lebertransplantation zur Verfügung. Es sind zwar sogenannte Bioreaktoren mit zumindest teilweise die Synthesefunktion normaler Leberzellen übernehmenden Zellen bekannt, doch sind diese derzeit nur experimentell einsetzbar und weisen noch keine ausreichende Funktion auf. Bei ca. 20 % der Patienten mit akutem Leberversagen werden Lebertransplantationen aus dem Grund vorgenommen, dass kein adäquates Verfahren zur Übernahme der Entgiftungsfunktion besteht und damit die Zeit bis zur Erholung der Leberfunktion nicht überbrückt werden kann.

Proteingebundene Substanzen dürften in der Pathogenese der hepatischen Enzephalopathie, des hepatischen Pruritus und des hepatorenalen Syndroms eine wichtige Rolle spielen. Zu diesen pathogenen Substanzen, die vorwiegend an Albumin gebunden sind, gehören insbesondere aromatische Verbindungen wie Phenolderivate, Indolderivate, Furanderivate oder aromatische Aminosäuren, Bilirubin, C₄-C₇-Carbonsäuren, Mercaptane, Digitoxin- und Benzodiazepinähnliche Substanzen sowie Metallkationen wie Kupferkationen, Aluminiumkationen oder Eisenkationen. Eine der wichtigsten Erkrankungen stellt dabei die hepatische Enzephalopathie dar, da sie vital lebensbedrohlich sein kann oder bleibende Schäden hinterlassen kann.

Um die Entgiftungsfunktion der Leber zu ersetzen, bestehen vielfältige Ansätze seit den siebziger Jahren, die sich vom Prinzip her an der Dialysetechnik orientieren:

### 1. Hämodialyse, Hämofiltration oder Hämodiafiltration

Diese konventionellen Verfahren werden derzeit bei vielen Patienten mit Leberversagen angewandt, da im letzten Stadium der Lebererkrankung in den meisten Fällen ein hepatorenales Syndrom auftritt, das auch zum dialysepflichtigen Nierenversagen führt. Durch Anwendung dieser Verfahren wird aber keine ausreichende Entgiftung proteingebundener Substanzen erzielt, sondern im wesentlichen nur die Eliminierung wasserlöslicher Substanzen mit niederem oder mittleren Molekulargewicht durchgeführt.

### 2. Hämoperfusion

Bei diesem Verfahren wird Blut oder Plasma über einen Adsorber geleitet (Kohle und/oder Kationen- oder Anionenaustauscher), um die proteingebundenen Toxine zu entfernen (O'Grady JG, Gimson AE, O'Brien CJ, Pucknell A, Hughes RD, Williams R: Controlled trials of charcoal hemoperfusion and prognostic factors in fulminant hepatic failure. Gastroenterology 94:1186-1192, 1988). Diese Methode birgt den Nachteil, dass sie unspezifisch ist und dass daher auch lebenswichtige Stoffe aus dem Blut oder Plasma entfernt werden.

### 3. Albumin als Adsorber mit zwei unterschiedlichen Verfahren

### A. MARS-System (Molecular Adsorbents Recirculating System)

Bei dem in der EP 0 615 780 beschriebenem MARS-System wird eine spezielle, mit Albumin beschichtete Dialysemembran verwendet. Die rezirkulierende albuminhaltige Dialysatflüssigkeit wird über 2 Adsorbersäulen (Kohle und Resin) geleitet, um die über Dialyse dem Patienten entfernten eiweißgebundenen Toxine zu eliminieren, und die Bindungsstellen des Albumins im Dialysat für Toxine bereitzustellen (Stange J, Hassanein TI, Mehta R, Mitzner SR, Bartlett RH: The molecular adsorbents recycling system as a liver support system based on albumin dialysis: a summary of preclinical investigations, prospective, randomized, controlled clinical trial, and clinical experience from 19 centers. Artif Organs 26:103-110, 2002).

### B. Albumindialyse

Die Albumindialyse ist ein der kontinuierlichen Hämodialyse verwandtes Verfahren. Ein Merkmal der kontinuierlichen Nierenersatztherapie ist die Verwendung langsamer Dialysatflüsse (1-2 l/h im Vergleich zu 30 l/h bei normaler Dialyse). Im Gegensatz zur klassischen kontinuierlichen Nierenersatztherapie wird bei der Albumindialyse das Dialysat mit Albumin versetzt, so dass eine 5% Lösung entsteht (Kreymann B, Seige M, Schweigart U, Kopp KF, Classen M: Albumin dialysis: effective removal of copper in a patient with fulminant Wilson disease and successful bridging to liver transplantation: a new possibility for the elimination of protein-bound toxins. J Hepatol 31:1080-1085, 1999). Die Verwendung von Albumin beruht darauf, dass es das hauptsächliche Trägerprotein für eiweißgebundene Toxine im Blut darstellt.

Den vorstehend beschriebenen Albumin-basierenden Systemen sind für den Einsatz bei großen Patientenzahlen hohe Therapiekosten gemeinsam (Tagestherapiekosten beim MARS-System oder Albumindialyse liegen derzeit bei ca. 2500 Euro). Darüber weisen diese Systeme eine unbefriedigende Entgiftungseffizienz auf: im Durchschnitt nur eine ca. 10-30 %-ige Reduktion des Bilirubinspiegels als Marker für proteingebundene Substanzen. Zwar führen die Albumin-basierenden Dialyseverfahren zu einer Verbesserung der Symptome der hepatischen Enzephalopathie, jedoch ist eine Normalisierung der Werte auch in Folge der hohen Therapiekosten nicht erreichbar.

Es ist daher Aufgabe der vorliegenden Erfindung, eine Dialysevorrichtung und ein Verfahren zur Dialyse bereitzustellen, mit dem in einem Schritt neben anderen unerwünschten dialysierbaren Substanzen proteingebundene Substanzen einfach und kostengünstig aus einer biologischen Flüssigkeit entfernt werden können.

Erfindungsgemäß wird diese Aufgabe durch eine Vorrichtung gemäß Patentanspruch 1 und ein Verfahren gemäß den Patentansprüchen 14 und 15 gelöst.

Die erfindungsgemäße Aufgabenlösung besteht darin, dass in einem Dialysierflüssigkeitskreislauf, in einem Kreislauf der biologischen Flüssigkeit oder auch in beiden Kreisläufen über wenigstens ein Mittel zum Inlösungbringen von zu entfernenden proteinbindenden Substanzen diese zumindest zwischenzeitlich in Lösung gebracht werden, was über eine Erniedrigung der Bindungsaffinität eines Trägerproteins resp. Adsorbers zu den zu entfernenden, proteinbindenden Substanzen erfolgt.

Diese Lösung weist den Vorteil auf, dass die zu entfernenden unerwünschten Substanzen von dem Trägerprotein und/oder Adsorber zumindest zeitweise in Lösung gebracht werden und anschließend leicht und effizient aus der biologischen Flüssigkeit entfernt werden können. So ist nicht nur eine schnelle Dialyse möglich, was einen erheblichen Kostenvorteil mit sich bringt, sondern auch eine besonders umfassende Reinigung der biologischen Flüssigkeit erzielbar.

Im Fall der Reinigung von Blut oder Blutplasma führt dies darüber hinaus zu einer patientenfreundlicheren Behandlungsweise, die im Bereich der Notfallmedizin bei akut lebensbedrohenden Zuständen von entscheidender Bedeutung für den Behandlungserfolg sein kann.

Das Mittel zum Inlösungbringen von zu entfernenden proteinbindenden Substanzen umfasst eine Einrichtung zur Einstellung des pH-Werts der verwendbaren Flüssigkeiten, eine Einrichtung zur Einstellung der Temperatur der verwendbaren Flüssigkeiten, eine Einrichtung zur Zugabe von Substituat zur Verdünnung oder Veränderung des Salzgehalts der verwendbaren Flüssigkeiten, eine Einrichtung zur Zugabe von dialysierbaren, an die zu entfernenden Substanzen bindenden Verbindungen und eine Einrichtung zur Bestrahlung mit Wellen der verwendbaren Flüssigkeiten. Im Rahmen der vorliegenden Erfindung können die unterschiedlichen Einrichtungen beliebig miteinander kombiniert werden. Bevorzugt werden wenigstens eine Einrichtung zur Einstellung des pH-Werts und wenigstens eine Einrichtung zur Einstellung der Temperatur in einem Kreislaufsystem verwendet.

Ein Vorteil der vorliegenden Erfindung liegt darin, dass mit einfachen Mitteln, d.h. konventionellen Einrichtungen zur Zugabe von Lösungen von Säuren, Basen, Substituat oder dialysierbaren Substanzen oder konventionellen Heiz-, Kühloder Ultraschallgeräten, die zu entfernenden Substanzen einfach und kostengünstig in Lösung gebracht werden können, über eine Schwächung der Bindung der proteinbindenden Substanzen an Trägerproteine resp. Adsorber. Die gelösten Substanzen (Toxine) sind dialysierbar und somit einfach entfernbar.

Es wurde nämlich gefunden, dass die Bindungsaffinität von Trägerproteinen wie Albumin resp. Adsorbern zu Toxinen durch einige Maßnahmen selektiv erniedrigt werden kann, was dazu führt, dass diese in Lösung gehen. Die proteingebundenen Substanzen in der biologischen Flüssigkeit oder in der Dialysierflüssigkeit stehen nämlich im Gleichgewicht zu einer geringen Menge nicht gebundener Substanzen. Durch Erniedrigung der Bindungsaffinität kann der Pool der nicht gebundenen, dialysierbaren Substanzen erhöht werden, da die freien Substanzen in Lösung gehen.

Zu diesen Maßnahmen gehören Temperaturerhöhung der Flüssigkeit, die gebundene Toxine enthält, Änderung des pH-Werts (saurer und basischer Bereich), eine Bestrahlung mit Wellen, Verdünnung der Flüssigkeit, Veränderung des Salzgehaltes und die Zuführung dialysierbarer Substanzen, die an die Toxine binden. Im Rahmen dieser Erfindung werden unter gelösten Substanzen sowohl solche verstanden, die als Einzelmoleküle solubilisiert vorliegen, wie auch solche, die an dialysierbare Substanzen gebunden sind, wobei der dadurch gebildete Komplex ebenfalls dialysierbar ist. Im Gegensatz dazu handelt es sich bei den gebundenen Substanzen um Komplexe mit hochmolekularen Trägerproteinen resp. Adsorbern, die nicht dialysierbar sind und in der Flüssigkeit praktisch dispergiert sind.

Wenn im Dialysierflüssigkeitskreislauf wenigstens ein erfindungsgemäßes Mittel zum Inlösungbringen von zu entfernenden proteinbindenden Substanzen vorgesehen ist, so sollte die in der Vorrichtung verwendbare Dialysierflüssigkeit einen Adsorber für die aus der biologischen Flüssigkeit zu entfernenden Substanzen enthalten. Ein kleiner Teil der proteinbindenden Toxine in der biologischen Flüssigkeit liegt in Lösung vor, und dieser kann durch die semipermeable Membran im Dialysator diffundieren und an die freien Bindungsstellen des Adsorbers in der Dialysierflüssigkeit binden. Anschließend wird über ein erfindungsgemäßes Mittel zum Inlösungbringen wie beispielsweise einer Einrichtung zur Zugabe von Säure die Bindungsaffinität zwischen Adsorber und Toxin zumindest zwischenzeitlich vermindert, wodurch die zu entfernenden Substanzen in Lösung gelangen und über Dialyse (Diffussion) oder Filtration (Konvektion)oder eine Kombination beider Verfahren, im folgenden Diafiltration genannt, aus dem Dialysierkreislauf entfernt werden können.

Auch im Kreislauf der biologischen Flüssigkeit, die an ein Trägerprotein gebundene, zu entfernende Substanzen enthält, kann wenigstens ein erfindungsgemäßes Mittel zum Inlösungbringen von zu entfernenden proteinbindenden Substanzen vorgesehen sein. So bewirkt beispielsweise die Einstellung einer erhöhten Temperatur, dass die Bindungsaffinität des Trägerproteins erniedrigt wird und die Substanzen in Lösung gehen. Dadurch können die gelösten, freien Substanzen dialysiert werden.

Bevorzugt enthält sowohl der Kreislauf der biologischen Flüssigkeit als auch der Dialysierflüssigkeitskreislauf wenigstens ein erfindungsgemäßes Mittel zum Inlösungbringen von zu entfernenden proteinbindenden Substanzen.

Ein Vorteil der Erfindung besteht darin, dass durch die verschiedenen zur Verfügung stehen Mittel Substanzen mit unterschiedlichem Bindungsverhalten entfernt werden können, insbesondere wenn verschiedene Maßnahmen miteinander kombiniert werden.

Ein besonders effizientes und kostengünstiges Mittel stellt die Einrichtung zur Einstellung des pH-Werts der Flüssigkeiten dar. Durch Einstellung des pH-Werts der Flüssigkeiten im sauren und/oder basischen Bereich kann selektiv die Bindung unterschiedlicher Substanzen zu den Trägerproteinen resp. Adsorbern beeinflusst werden. So kann durch Zugabe einer Säure der pH-Wert der Flüssigkeit abgesenkt werden, wodurch die Bindung von gewissen Toxinen an Proteine im sauren Bereich vermindert wird und damit der Pool von freien Toxinen in der Flüssigkeit erhöht wird. Beispielsweise kann derart die Bindung von Kupferionen an Albumin geschwächt werden, so dass freie, gelöste Kupferionen im darauf folgenden Filter durch Dialyse, Filtration oder Diafiltration entfernt werden können. Analog dazu kann der pH-Wert der Flüssigkeit im basischen Bereich eingestellt werden, um die im alkalischen Bereich freigesetzten Toxine anschließend durch Dialyse, Filtration oder Diafiltration aus der Flüssigkeit zu eliminieren.

Um die Bindungsfähigkeit der verwendbaren Adsorber wieder herzustellen, wird der pH-Wert der Dialysierflüssigkeit anschließend in den für die Bindung von toxischen Substanzen an Adsorber optimalen Bereich abgesenkt, was die Rezyklierung der Dialysierflüssigkeit erlaubt. Somit weist die vorliegende Erfindung über die einfache Rezyklierung des Adsorbers, insbesondere bei Verwendung von Albumin, einen entscheidenden Kostenvorteil auf.

Bevorzugt enthält ein Kreislaufsystem (für Dialysierflüssigkeit oder zu reinigende biologische Flüssigkeit) zwei Einrichtungen zur Einstellung des pH-Werts, ganz besonders bevorzugt drei Einrichtungen zur Einstellung des pH-Werts, um mit der ersten Einrichtung den pH-Wert im sauren resp. basischen Bereich, mit der zweiten im basischen resp. saurem Bereich und mit der dritten Einrichtung den pH-Wert wieder im ursprünglichen Bereich (in der Regel neutral) einzustellen. Weiterhin bevorzugt enthält ein Kreislaufsystem (für Dialysierflüssigkeit oder zu reinigende biologische Flüssigkeit) zwei Einrichtungen zur Einstellung der Temperatur, so dass die verwendbare Flüssigkeit beispielsweise erwärmt und anschließend durch Kühlen wieder auf die vorherige Temperatur oder die jeweils erwünschte Temperatur gebracht werden kann. Ganz besonders bevorzugt enthält ein Kreislaufsystem drei Einrichtungen zur Einstellung des pH-Werts und zwei Einrichtungen zur Einstellung der Temperatur.

Ein weiterer Vorteil der Erfindung besteht darin, dass mittels im Dialysierflüssigkeitskreislauf und/oder im Kreislauf der biologischen Flüssigkeit vorgesehener Einrichtungen zur Dialyse, Filtration oder Diafiltration die gelösten, dialysierbaren Substanzen aus den Flüssigkeiten (Dialysierflüssigkeit oder zu reinigende biologische Flüssigkeit) nach Ablösung von den Trägerproteinen resp. Adsorbern leicht und effizient entfernt werden können. Dabei können konventionelle Dialysegeräte, wie sie dem Fachmann bekannt sind, verwendet werden. Zusätzlich werden bevorzugt Vorrichtungen zur Veränderung der pH-/Temperaturwerte und Vorrichtungen zur entsprechenden Kontrolle dieser Veränderungen eingesetzt. Vorteilhafterweise wird in der erfindungsgemäßen Vorrichtung hinter eine Einrichtung zur Einstellung des pH-Werts/Temperatur der zu verwendenden Flüssigkeit eine Einrichtung zur Dialyse, Filtration oder Diafiltration geschaltet, um die freien, gelösten Substanzen direkt aus der Flüssigkeit zu entfernen. Besonders bevorzugt ist eine Vorrichtung, bei der im Dialysierflüssigkeitskreislauf und/oder im Kreislauf der biologischen Flüssigkeit in Folge eine Einrichtung zur Zugabe von Säure oder Base, eine Einrichtung zur Dialyse oder Diafiltration, eine Einrichtung zur Zugabe von Base oder Säure, eine Einrichtung zur Dialyse, Filtration oder Diafiltration und eine Einrichtung zur Zugabe von Säure oder Base vorgesehen sind. Dadurch können unterschiedliche proteinbindende Substanzen sehr effizient aus der Dialysierflüssigkeit und der biologischen Flüssigkeit entfernt werden, und die gereinigte Dialysierflüssigkeit kann wiederum dem Dialysator zugeführt werden, um den Adsorber wiederum mit proteinbindenden Substanzen zu beladen.

Ein Vorteil einer besonderen Ausführungsform der vorliegenden Erfindung, bei der nur im Kreislauf der biologischen Flüssigkeit ein Mittel zum Inlösungbringen wie beispielsweise eine Einrichtung zur Einstellung des pH-Werts angeordnet ist, besteht darin, dass zur Entfernung unerwünschter proteingebundener Toxine die Dialysierflüssigkeit keinen Adsorber, wie beispielsweise das Akzeptorprotein Albumin, enthalten muss, was zu einer drastischen Senkung der Dialysekosten führt.

Unter Einrichtungen zur Einstellung des pH-Werts fallen insbesondere Einrichtungen zur Zugabe von Säure oder Base wie beispielsweise Dosierpumpen. Als Säuren oder Basen bieten sich wässrige Lösungen von biologisch verträglichen Säuren oder Basen an. Allgemein bevorzugt werden Säuren oder Basen eingesetzt, deren konjugierte Basen oder Säuren im menschlichen Organismus natürlich auftretende Ionen darstellen. Beispielsweise können als Säuren Salzsäure, Schwefelsäure oder Essigsäure verwendet werden, bevorzugt Salzsäure. Als Basen können beispielsweise Natronlauge oder Kalilauge eingesetzt werden, bevorzugt Natronlauge. Die biologische oder Dialyse-Flüssigkeit kann beispielsweise durch Zugabe von Säure auf einen pH-Wert zwischen 2,5 und 7, und durch Zugabe von Base auf einen pH-Wert zwischen 7 und 12,5 eingestellt werden. Der jeweils gewünschte pH-Wert richtet sich im wesentlichen nach der Beschaffenheit der verwendeten Flüssigkeit und der zu entfernenden Substanzen.

Unter Einrichtung zur Einstellung der Temperatur fallen insbesondere Einrichtungen zum Erwärmen wie übliche Heizgeräte, Mikrowellengeräte oder auch Infrarotgeräte oder konventionelle Kühlaggregate als Einrichtung zum Kühlen. Im Dialysierflüssigkeitskreislauf und/oder der Kreislauf der biologischen Flüssigkeit können jeweils eine oder mehrere Einrichtungen zum Erwärmen/Kühlen angeordnet sein. Insbesondere können durch Erwärmung der verwendbaren Flüssigkeiten die zu entfernenden Substanzen in Lösung gebracht werden, während mittels Kühlen die biologische Flüssigkeit oder die Dialysierflüssigkeit wieder auf die gewünschte Temperaturen gebracht werden können.

Ein weiterer Vorteil der Erfindung besteht darin, dass durch eine Einrichtung zum Kühlen der verwendbaren Dialysierflüssigkeit die Bindungsaffinität des Adsorbers selektiv erhöht werden kann, wodurch freie, gelöste Substanzen, die in die Dialyseflüssigkeit diffundiert sind, durch rezyklierte Adsorber gebunden werden können.

Die gewünschte Temperatur der zu verwendenden Flüssigkeiten hängt im wesentlichen von deren Beschaffenheit ab. Wird als biologische Flüssigkeit Blut oder Blutplasma eingesetzt, so kann auf eine Temperatur bis max. 42 °C, bevorzugt 39 bis 41 °C erwärmt werden. Bei Verwendung der erfindungsgemäßen Dialysevorrichtung in einem extrakorporalen Kreislauf an einem Patienten kann die Temperatur wieder auf einen für den Patienten optimalen Temperaturwert im Bereich von 35 bis 37 °C, bei Patienten mit hepatischer Enzephalopathie ca. 35 ° Celsius, herabgesenkt werden.

Die Erwärmung der zu verwendenden Flüssigkeit im Kreislaufsystem kann über eine direkte Erwärmung des flüssigkeitsgefüllten Schlauchsystems mittels eines Heizgeräts oder mittels Bestrahlung mit Mikrowellen oder Infrarot durchgeführt werden. Aufgrund des Wärmeaustauschs zwischen der zu reinigenden Flüssigkeit und der Dialysierflüssigkeit im Dialysator kann es beispielweise ausreichend sein, nur im Dialysierflüssigkeitskreislauf Einrichtungen zum Erwärmen zu enthalten, wobei trotzdem eine Erwärmung der biologischen Flüssigkeit auftritt. Gemäß einer weiteren Ausführungsform kann eine Einrichtung zum Erwärmen auch vor dem Eintritt in den Dialysierflüssigkeitskreislauf oder den Kreislauf der biologischen Flüssigkeit einer Einrichtung zur Einstellung des pH-Werts oder einer Einrichtung zur Zugabe von Substituat nachgeschaltet ist. In diesem Fall werden die Dialysierflüssigkeit und/oder die zu reinigende Flüssigkeit durch Zugabe von angewärmten Lösungen erwärmt.

Als Einrichtungen zur Bestrahlung mit Wellen kann beispielsweise ein Ultraschallgerät verwendet werden.

Als Mittel zum Inlösungbringen bietet sich auch eine Einrichtung zur Zugabe von dialysierbaren, an die zu entfernenden Substanzen bindenden Verbindungen. Dabei kann es sich um konventionelle Dosierpumpen handeln, die wässrige Lösungen der dialysierbaren Verbindungen zuführen. Die dialysierbaren Substanzen, die zum Teil toxingebunden vorliegen, können über die konventionellen Einrichtungen zur Dialyse oder Diafiltration einfach entfernt werden. Als dialysierbare Verbindungen bieten sich allgemein Verbindungen an, die dialysierbar sind, d.h. mit niedrigem/mittleren Molekulargewicht, und sich durch eine starke Affinität zu den zu entfernenden Substanzen auszeichnen. Zu den bevorzugten Verbindungen gehören Coffein, das an Bilirubin bindet, und gängige Chelatbildner wie Penicillamin, Trientine, Deferoxamin, Preferiprone, HBED, Vitamin C, BAL, DMPS oder DMSA, die an Metallkationen wie beispielsweise Kupferionen oder Eisenionen binden. Die dialysierbaren Verbindungen können sowohl der biologischen Flüssigkeit als auch der Dialysierflüssigkeit zugefügt werden, bevorzugt erfolgt die Zugabe aber in die Dialysierflüssigkeit, um Komplikationen durch mögliche Verunreinigungen der biologischen Flüssigkeit bei unvollständiger Entfernung durch Dialyse zu vermeiden.

Zu einer Einrichtung zur Zugabe von Substituat zur Verdünnung oder Veränderung des Salzgehalts der verwendbaren Flüssigkeiten gehören übliche Dosierpumpen, mit denen eine Substituatlösung zugegeben werden kann. Bevorzugt solch eine Einrichtung in Kombination mit einer Einrichtung zum Erwärmen, die dieser nachgeschaltet ist, verwendet, so dass in den Kreislauf der verwendeten Flüssigkeiten angewärmtes Substituat zugegeben wird. Als Substituatlösung kommen wässrige Lösungen, die verschiedene Salze sowie auch Harnstoff enthalten können, in Frage. Dabei kann es sich um kommerzielle Dialysierflüssigkeiten handeln, die je nach Bedarf durch Zufügen von Salzen auf die erwünschte Konzentration eingestellt werden können. Das Substituat dient nicht nur dazu, die zu entfernenden Substanzen durch Veränderung der Salzkonzentration in der Flüssigkeit in Lösung zu bringen. Die Salzkonzentration der biologischen Flüssigkeit, z.B. Blut, kann durch Substituatzugabe auch genau dem Zustand des Patienten entsprechend eingestellt werden. Zudem kann dadurch im Dialysierkreislauf die Bindungsfähigkeit von rezykliertem Adsorber für Toxine wiederhergestellt werden. Zusätzlich kann die Zugabe von Harnstoff für eine bessere Bindungsfähigkeit des Adsorbers notwendig sein.

Als Dialysator können konventionelle, zurzeit z.B. für die Hämodialyse verwendete Dialysatoren eingesetzt werden. Es ist aber auch vorstellbar, dass Membranen mit größeren Poren als derzeit für die Dialyse verwendet werden, in Frage kommen. Der Dialysator ist mit einer konventionellen, semipermeablen Dialysemembran ausgestattet, gegebenenfalls kann die Diffusion durch die Membran durch konvektiven Transport mittels Filtration unterstützt werden. Im wesentlichen umfasst der Dialysator zwei Kammern, die durch eine Dialysemembran getrennt sind, an die jeweils ein Kreislaufsystem (Schlauchsystem) für die zu verwendenden Flüssigkeiten angeschlossen ist. Die zu reinigende biologische Flüssigkeit und die Dialysierflüssigkeit werden üblicherweise im Gegenstromverfahren geführt. Möglich ist aber auch die Durchführung eines Gleichstromverfahrens. Übliche Ausgestaltungen eines Dialysegeräts wie Manometer, Luftdetektoren, Pumpeneinrichtungen wie Heparinpumpe, Blutpumpen usw. werden von der erfindungsgemäßen Vorrichtung mitumfasst. Mit der erfindungsgemäßen Vorrichtung können sowohl langsame (1-2 l/h) als auch normale Dialysatflüsse (25-50 l/h) erzielt werden, wie auch Geschwindigkeiten dazwischen je nach Bedarf.

Zu den biologischen Flüssigkeiten, die in der erfindungsgemäßen Vorrichtung resp. im erfindungsgemäßen Verfahren verwendet werden können, gehören alle Körperflüssigkeiten von Menschen oder Tieren, insbesondere Blut oder Blutplasma, besonders bevorzugt humaner Abstammung. Neben der Entfernung von proteingebundenen Substanzen aus den verwendeten biologischen Flüssigkeiten erfolgt gleichzeitig eine Eliminierung üblicherweise bei der konventionellen Dialyse entfernbarer, wasserlöslicher Substanzen wie beispielsweise Harnstoff oder verschiedene Ionen. Die zu entfernenden proteinbindenden Substanzen sind bevorzugt an das Trägerprotein Albumin gebunden. Die erfindungsgemäße Vorrichtung eignet sich insbesondere zur Blut- und Plasmareinigung im Bereich der Medizin, und kann sowohl im Bereich der Blutkonservenverarbeitung wie auch bei extrakorporaler Dialyse an Patienten eingesetzt werden.

Als Dialysierflüssigkeiten können konventionelle Dialysierflüssigkeiten, wie sie dem Fachmann bekannt sind, verwendet werden. Die Ionenkonzentration kann jeweils an den Bedarf des Patienten angepasst werden. Je nach Bedarf können gebräuchliche ionenhaltige wässrige Lösungen oder reines Wasser verwendet werden. Gegebenenfalls werden die konventionellen Dialysierflüssigkeiten mit einem Adsorber für die zu entfernenden proteinbindenden Substanzen versehen. Als Adsorber kommen beispielsweise Resinat und Akzeptorproteine in Frage. Ein bevorzugtes Akzeptorprotein ist Albumin, dieses kann humanes Serumalbumin, tierisches Albumin oder auch gentechnisch hergestelltes Albumin sein. Besonders bevorzugt wird humanes Serumalbumin eingesetzt. Die Serumalbumin-Lösungen können gegebenenfalls mit Wasser oder konventionellen Dialysierflüssigkeiten verdünnt werden. Die verwendete Dialysierflüssigkeit kann humanes Serumalbumin in einer Konzentration von 1 bis 25 g pro 100 ml, bevorzugt 2 bis 10 g pro 100 ml, besonders bevorzugt 4 bis 6 g pro 100 ml, enthalten.
Des weiteren können als Dialysierflüssigkeit auch Blut, Blutserum oder fresh frozen plasma verwendet werden. Bei der Dialysierflüssigkeit kann es sich auch um Dialysat aus einem Bioreaktor handeln. Derzeit sind für Bioreaktoren (mit lebenden Leberzellen arbeitende Systeme zur Leberersatztherapie) enorme Blutmengen notwendig; so muss dem Patienten während der Zirkulation des Bioreaktors bis zu einem Liter Blut aus dem Kreislauf entnommen werden. Zur Anregung der Synthesefunktion der Leberzellen des Bioreaktors kann aber auch ein System ausreichend sein, bei dem ein Dialysat, das Substanzen enthält, die normalerweise in der Leber entgiftet werden, zur Anregung eingesetzt wird. Entsprechend kann ein wie unter 1 und 2 beschriebenes Dialysat zunächst im extrakorporalen Kreislauf über den Bioreaktor geleitet werden. Dieses Dialysat wird dann wie unter 2 beschrieben gereinigt und das Dialysat dem Patienten wieder zugeführt. Hierzu kann es notwendig sein, dem Dialysat kontinuierlich Albumin zuzuführen oder aber eine Kapillare resp. eine Membran zu benutzen, die für Albumin durchlässiger ist als derzeit verwendete Dialysefilter.

Die erfindungsgemäße Vorrichtung kann mit einem oder mehreren konventionellen pH-Messgeräten und/oder Temperaturmeßgeräten ausgestattet sein zur Überwachung der entsprechenden Eigenschaften der verwendeten Flüssigkeiten.

Die Erfindung betrifft weiterhin ein Verfahren zur Entfernung von unerwünschten Substanzen aus einer biologischen Flüssigkeit umfassend das Dialysieren einer biologischen Flüssigkeit gegen eine Dialysierflüssigkeit durch eine semipermeable Membran, dadurch gekennzeichnet, dass die Dialysierflüssigkeit einen Adsorber für zu entfernende proteinbindende Substanzen enthält, und dass die Dialysierflüssigkeit durch Zugabe von Säure, Base oder dialysierbarer Substanzen, durch Verdünnung, Veränderung des Salzgehalts, Bestrahlung mit Wellen oder Erwärmung so eingestellt wird, dass die Bindungsaffinität des Adsorbers zu den gebundenen Substanzen zumindest zwischenzeitlich erniedrigt wird und die zu entfernenden Substanzen in Lösung gehen. Des weiteren wird ein Verfahren bereitgestellt zur Entfernung von unerwünschten Substanzen aus einer biologischen Flüssigkeit umfassend das Dialysieren einer biologischen Flüssigkeit gegen eine Dialysierflüssigkeit durch eine semipermeable Membran,
dadurch gekennzeichnet, dass die biologische Flüssigkeit durch Zugabe von Säure, Base oder dialysierbarer Substanzen, durch Verdünnung, Veränderung des Salzgehalts, Bestrahlung mit Wellen oder Erwärmung so eingestellt wird, dass die Bindungsaffinität des Trägerproteins zu den gebundenen, zu entfernenden Substanzen erniedrigt wird und die zu entfernenden Substanzen in Lösung gehen.

Die erfindungsgemäßen Verfahren und Vorrichtungen können allgemein zur Reinigung biologischer Flüssigkeiten verwendet werden. Zu biologischen Flüssigkeiten gehören alle Körperflüssigkeiten von Menschen oder Tieren, insbesondere Blut oder Blutplasma, besonders bevorzugt humaner Abstammung. Dabei ist es möglich, die entnommenen Flüssigkeiten, insbesondere Blut oder Blutplasma, dem Körper wieder zurückzuführen, oder für andere Zwecke zugänglich zu machen. So können beispielsweise Blutkonserven gereinigt werden, oder die gereinigten biologischen Flüssigkeiten werden weiteren kommerziellen Zwecken oder Forschungszwecken zugeführt.

Zur Durchführung der erfindungsgemäßen Verfahren ist die vorstehend beschriebene Vorrichtung geeignet. Weitere Einzelheiten, Merkmale und Vorteile des Verfahrens ergeben sich aus der vorstehenden Besprechung der Vorrichtung und den Ansprüchen.

Nachfolgend werden unter Bezugnahme auf die Figuren drei Ausführungsbeispiele der Erfindung näher erläutert. Die Figuren zeigen besondere Ausführungsformen der erfindungsgemäßen Vorrichtung in schematischer Darstellung.

### Es zeigen:

Figur 1 eine vereinfachte schematische Darstellung einer Ausführungsform der erfindungsgemäßen Vorrichtung mit Einrichtungen zum Erwärmen und Kühlen und einer Einrichtung zur Substituatzugabe im extrakorporalem Kreislauf.

Figur 2 eine vereinfachte schematische Darstellung einer Ausführungsform der erfindungsgemäßen Vorrichtung mit Einrichtungen zur Einstellung des pH-Werts im extrakorporalem Kreislauf.
Figur 3 eine vereinfachte schematische Darstellung einer Ausführungsform der erfindungsgemäßen Vorrichtung mit Einrichtungen zum Erwärmen und Kühlen, Einrichtungen zur Einstellung des pH-Werts und einer Einrichtung zur Substituatzugabe im Dialysierflüssigkeitskreislauf.

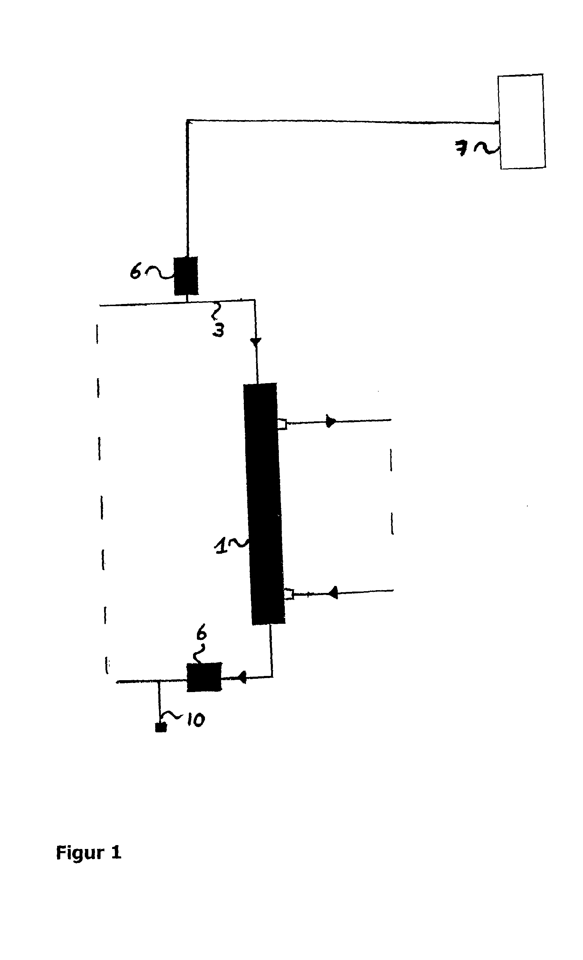

Figur 1 zeigt eine Vorrichtung zur Hämodialyse bestehend im wesentlichen aus einem Dialysator (1), einem Dialysierflüssigkeitskreislauf (2) (in der Figur nur angedeutet, bei dieser Ausführungsform muss verbrauchtes Dialysat nicht rezykliert werden), einem Blutkreislauf (3) (in der Figur nur andeutungsweise eingezeichnet), Heiz- und Kühlgeräten (6), einer Einrichtung zur Substituatzugabe (7) und einem Temperaturmessgerät (10).
Das Blut wird im Blutkreislauf (3) vor Eintritt in den Dialysator (1) über die Einrichtung (7) mit im Heizgerät (6) angewärmten Substituat aus z.B. konventioneller Hämofiltrationslösung versetzt. Anschliessend gelangt das angewärmte Blut in die Blutkammer des Dialysators (1). Aufgrund der erhöhten Temperatur des Bluts kommt es zu einer erhöhten Freisetzung von proteingebundenen Substanzen von den Trägerproteinen, und somit liegt ein erhöhter pool gelöster, dialysierbarer Toxine vor, die über die Dialysemembran in die Dialysekammer des Dialysators (1) diffundieren. Nachdem das von proteingebundenen Substanzen gereinigte Blut den Dialysator (1) verlassen hat, wird es durch das Kühlaggregat (6) wieder abgekühlt auf eine physiologisch annehmbare Temperatur, die mittels dem Temperaturmessgerät (10) überprüft wird. Alternativ kann die Bluttemperatur und damit die Temperatur des Patienten auch durch die Steuerung der Dialysattemperatur erfolgen. Anschließend wird das Blut im Blutkreislauf (3) zurückgeführt.

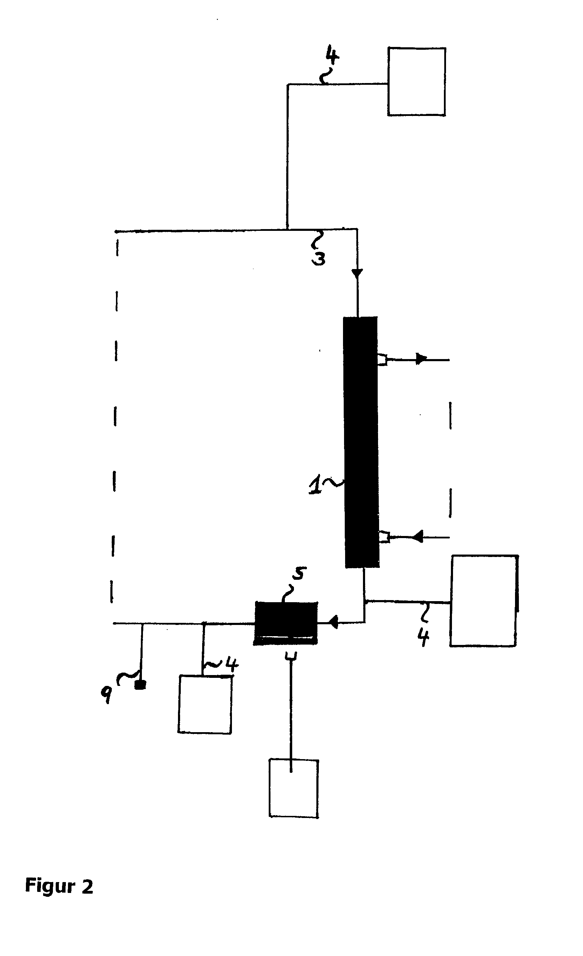

Figur 2 zeigt eine Vorrichtung zur Hämodialyse bestehend im wesentlichen aus einem Dialysator (1), einem Dialysierflüssigkeitskreislauf (2) (in der Figur nur angedeutet, bei dieser Ausführungsform muss verbrauchtes Dialysat nicht rezykliert werden), einem Blutkreislauf (3) (in der Figur nur andeutungsweise eingezeichnet), Dosierpumpen zur Säure- oder Basenzugabe (4), Dialysator (5) und ein pH-Messgerät (11).
Das Blut wird im Blutkreislauf (3) vor Eintritt in den Dialysator (1) mittels der Dosierpumpe (4) mit HCl-Lösung versetzt. Dadurch wird der pH-Wert des Bluts herabgesenkt, und ein Teil der Toxine gehen in Lösung. Anschließend gelangt das angesäuerte Blut in die Blutkammer des Dialysators (1). Die gelösten dialysierbaren Substanzen können über die Dialysemembran in die Dialysekammer des Dialysators (1) diffundieren. Nachdem das teilweise von proteingebundenen Substanzen befreite Blut den Dialysator (1) verlassen hat, wird es mittels der Dosierpumpe (4) mit NaOH-Lösung versetzt, wodurch der pH-Wert im basischen Bereich eingestellt wird, und weitere proteinbindende Toxine in Lösung gehen. Stromabwärts gelangt das Blut in ein weiteren Dialysator (5). Dort wird erneut entweder eine Dialyse, Filtration oder eine Diafiltration durchgeführt um im alkalischen Bereich gelöste, sonst proteingebundene, Substanzen zu eliminieren. Anschließend wird der pH-Wert über eine Dosierpumpe (4) mit HCl-Lösung im neutralen Bereich bei ca. 7.4 eingestellt, was mittels dem pH-Messgerät (11) überprüft wird. Daraufhin wird das Blut im Blutkreislauf (3) zurückgeführt.

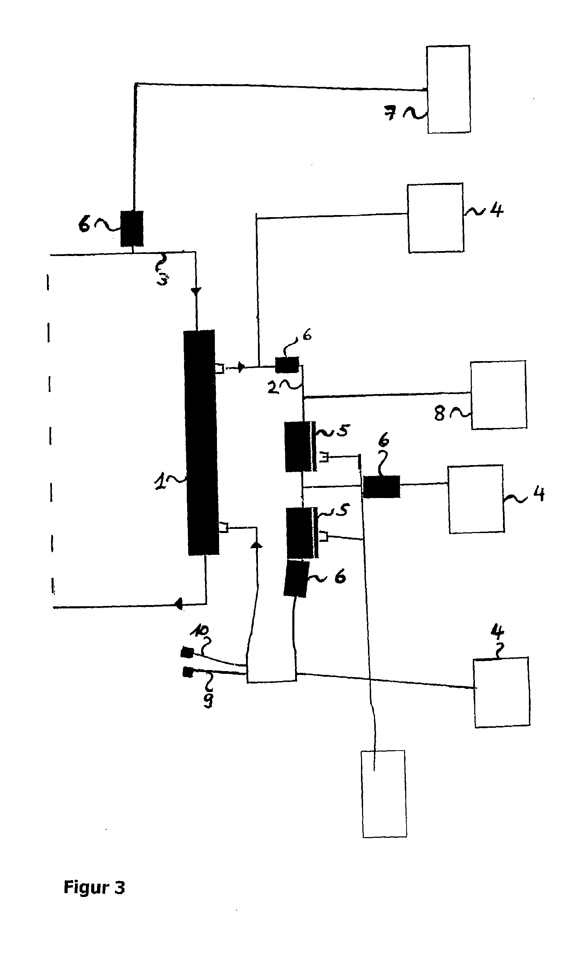

Figur 3 zeigt eine Vorrichtung zur Hämodialyse bestehend im wesentlichen aus einem Dialysator (1), einem Dialysierflüssigkeitskreislauf (2), einem Blutkreislauf (3) (in der Figur nur andeutungsweise eingezeichnet), Dosierpumpen zur Säure- oder Basenzugabe (4), Dialysatoren (5), Heiz- und Kühlgeräten (6), Einrichtung zur Substituatzugabe (7), Einrichtung zur Coffeinzugabe (8), pH-Messgerät (11) und Temperaturmessgerät (10).

Das Blut wird im Blutkreislauf (3) vor Eintritt in den Dialysator (1) über die Einrichtung (7) mit im Heizgerät (6) angewärmten Substituat aus z.B. Hämofiltrationslösung versetzt. Anschliessend gelangt das angewärmte Blut in die Blutkammer des Dialysators (1). Aufgrund der erhöhten Temperatur des Bluts liegt ein erhöhter pool freier, dialysierbarer Toxine vor, die über die Dialysemembran in die Dialysekammer des Dialysators (1) diffundieren. Zudem enthält die Dialysierflüssigkeit Albumin, das an die Toxine bindet, so dass der pool freier Substanzen in der Dialysierflüssigkeit niedrig gehalten wird, wodurch die Diffusion der Toxine in die Dialysierflüssigkeit verstärkt wird. Nachdem das von proteingebundenen Substanzen gereinigte Blut den Dialysator (1) verlassen hat, wird es im Blutkreislauf (3) zurückgeführt.

Die Dialysierflüssigkeit aus dem Dialysator (1), die albumingebundene Toxine enthält, gelangt in den Dialysierflüssigkeitskreislauf (2). Über die Dosierpumpe (4) wird der Dialysierflüssigkeit HCl-Lösung zugefügt. Dadurch wird der pH-Wert der Dialysierflüssigkeit herabgesenkt, und der pool gelöster, freier Toxine in der Flüssigkeit steigt an. Stromabwärts ist im Dialysierflüssigkeitskreislauf (2) ein Heizgerät (6) angeordnet, das die Dialysierflüssigkeit auf 41-45°C erwärmt, wodurch der pool freier Toxine weiter erhöht wird und der Anteil proteingebundener Toxine absinkt. Anschließend ist im Kreislaufsystem (2) eine Dosierpumpe (8) für Coffein angebracht. Durch die Zugabe von Coffein wird insbesondere Bilirubin gebunden, und dadurch erniedrigt sich der Anteil des proteingebundenen Bilirubins in der Dialysierflüssigkeit. Stromabwärts gelangt die Dialysierflüssigkeit in ein Dialysator (5). Dort werden dem System ein Teil der Dialysierflüssigkeit entzogen, um die Konzentration des Adsorbers im gewünschten Bereich zu halten. Zusätzlich wird durch Dialyse, Filtration oder Diafiltration eine Reinigung des Dialysates durchgeführt, insbesondere von freien, proteinbindende Substanzen und coffein-gebundenes Bilirubin. Das Albumin kann aufgrund seiner hohen Molmasse den Filter nicht passieren. Nach dem Austritt aus dem Dialysator (5) ist im Dialysierflüssigkeitskreislauf (2) eine Dosierpumpe (4) zur Zugabe von NaOH-Lösung angeordnet, wobei vor Eintritt in den Kreislauf ein Heizgerät (6) angeordnet ist. Stromabwärts folgt wiederum ein Dialysator (5), das dem System die zugeführte Flüssigkeit entzieht und im alkalischen Bereich gelöste Substanzen durch Dialyse, Filtration oder Diafiltration eliminiert. Anschliessend ist im Kreislaufsystem (2) eine Kühlvorrichtung (6) angebracht, mit der die Temperatur der Dialysierflüssigkeit entsprechend der gewünschten Patiententemperatur angepasst werden kann. Über die nachfolgende Dosierpumpe (4) wird der Dialysierflüssigkeit HCl-Lösung zugefügt, um den pH-Wert der Dialysierflüssigkeit im Neutralen einzustellen, und so die Bindungsfähigkeit von Albumin wieder erhöht wird, und keine negative Beeinflussung des pH-Werts des Blutes im Dialysator auftritt. Anschliessend sind im Kreislaufsystem (2) ein pH-Messgerät (11) und ein Temperaturmessgerät (10) angeordnet, um den pH-Wert und Temperaturwert der gereinigten Dialyseflüssigkeit vor Wiedereintritt in den Dialysator (1) zu überprüfen.

## Patentansprüche

1. Vorrichtung zur Dialyse einer biologischer Flüssigkeit umfassend einen Dialysator (1), und
- einen Dialysierflüssigkeitskreislauf (2), in dem wenigstens ein Mittel (4;6;7;8;9) zum Inlösungbringen von zu entfernenden proteinbindenden Substanzen und wenigstens eine Einrichtung (5) zur Dialyse, Filtration oder Diafiltration vorgesehen sind, wobei eine in der Vorrichtung verwendbare Dialysierflüssigkeit einen Adsorber für die aus der biologischen Flüssigkeit zu entfernenden Substanzen enthält,
und/oder
- einen Kreislauf (3) der biologischen Flüssigkeit, in dem wenigstens ein Mittel (4;6;7;8;9) zum Inlösungbringen von zu entfernenden proteingebundenen Substanzen vorgesehen ist, wobei eine in der Vorrichtung verwendbare biologischen Flüssigkeit proteingebundene Substanzen enthält.

2. Vorrichtung gemäß Anspruch 1, **dadurch gekennzeichnet, dass** das wenigstens eine Mittel (4;6;7;8;9) zum Inlösungbringen von zu entfernenden proteinbindenden Substanzen eine Einrichtung (4) zur Einstellung des pH-Werts der verwendbaren Flüssigkeiten und/oder eine Einrichtung (6) zur Einstellung der Temperatur der verwendbaren Flüssigkeiten und/oder eine Einrichtung (7) zur Zugabe von Substituat zur Verdünnung oder Veränderung des Salzgehalts der verwendbaren Flüssigkeiten und/oder eine Einrichtung (8) zur Zugabe von dialysierbaren, an die zu entfernenden Substanzen bindenden Verbindungen und oder eine Einrichtung (9) zur Bestrahlung mit Wellen der verwendbaren Flüssigkeiten umfaßt.

3. Vorrichtung gemäß Anspruch 2, **dadurch gekennzeichnet, dass** die Einrichtung (4) zur Einstellung des pH-Werts der verwendbaren Flüssigkeiten Einrichtungen zur Zugabe von Säure oder Base umfasst.

4. Vorrichtung gemäß Anspruch 2, **dadurch gekennzeichnet, dass** die Einrichtung (6) zur Einstellung der Temperatur Einrichtungen zum Erwärmen oder Kühlen umfasst.

5. Vorrichtung gemäß Anspruch 4, **dadurch gekennzeichnet, dass** die Einrichtung (6) zum Erwärmen ein Heizgerät, ein Mikrowellengerät oder ein Infrarotgerät umfasst und die Einrichtung (6) zum Kühlen ein Kühlaggregat darstellt.

6. Vorrichtung gemäß Anspruch 2, **dadurch gekennzeichnet, dass** die Einrichtung (9) zum Bestrahlen ein Ultraschallgerät umfasst.

7. Vorrichtung gemäß Anspruch einem der vorhergehenden Ansprüche, **dadurch gekennzeichnet, dass** im Kreislauf (3) der biologischen Flüssigkeit wenigstens eine weitere Einrichtung (5) zur Dialyse, Filtration oder Diafiltration vorgesehen ist.

8. Vorrichtung gemäß einem der vorhergehenden Ansprüche, **dadurch gekennzeichnet, dass** im Dialysierflüssigkeitskreislauf (2) und/oder im Kreislauf (3) der biologischen Flüssigkeit eine Einrichtung (6) zum Erwärmen und eine Einrichtung (6) zum Kühlen der verwendbaren Flüssigkeiten vorgesehen sind.

9. Vorrichtung gemäß einem der vorhergehenden Ansprüche, **dadurch gekennzeichnet, dass** im Dialysierflüssigkeitskreislauf (2) und/oder im Kreislauf (3) der biologischen Flüssigkeit als Mittel (4;6;7;8;9) zum Inlösungbringen von zu entfernenden proteinbindenden Substanzen wenigstens eine Einrichtung (4) zur Einstellung des pH-Werts und wenigstens eine Einrichtung (6) zum Erwärmen der verwendbaren Flüssigkeiten vorgesehen sind.

10. Vorrichtung gemäß einem der vorhergehenden Ansprüche, **dadurch gekennzeichnet, dass** der Dialysierflüssigkeitskreislauf (2) und/oder der Kreislauf (3) der biologischen Flüssigkeit drei Einrichtungen (4) zur Zugabe von Säure oder Base umfasst.

11. Vorrichtung gemäß einem der vorhergehenden Ansprüche, **dadurch gekennzeichnet, dass** der Dialysierflüssigkeitskreislauf (2) und/oder der Kreislauf (3) der biologischen Flüssigkeit in Folge von eine Einrichtung (4) zur Zugabe von Säure oder Base, eine Einrichtung (5) zur Dialyse, Filtration oder Diafiltration, eine Einrichtung (4) zur Zugabe von Base oder Säure, eine Einrichtung (5) zur Dialyse, Hämofiltration oder Diafiltration und eine Einrichtung (4) zur Zugabe von Säure oder Base enthält.

12. Vorrichtung gemäß einem der vorhergehenden Ansprüche, **dadurch gekennzeichnet, dass** die Einrichtung (6) zum Erwärmen vor dem Eintritt in den Dialysierflüssigkeitskreislauf (2) oder den Kreislauf (3) der biologischen Flüssigkeit einer Einrichtung (4) zur Einstellung des pH-Werts oder einer Einrichtung (7) zur Zugabe von Substituat nachgeschaltet ist.

13. Vorrichtung gemäß einem der vorhergehenden Ansprüche, **dadurch gekennzeichnet, dass** in dem Dialysierflüssigkeitskreislauf (2) und/oder dem Kreislauf (3) der biologischen Flüssigkeit eine oder mehrere pH-Messgeräte (11) und/oder Temperaturmessgeräte (10) enthalten sind.

14. Verfahren zur Entfernung von unerwünschten Substanzen aus einer biologischen Flüssigkeit, umfassend das Dialysieren einer biologischen Flüssigkeit gegen eine Dialysierflüssigkeit durch eine semipermeable Membran, **dadurch gekennzeichnet, dass** die Dialysierflüssigkeit einen Adsorber für zu entfernende proteinbindende Substanzen enthält, und dass die Dialysierflüssigkeit durch Zugabe von Säure, Base oder dialysierbarer Substanzen, durch Verdünnung, Veränderung des Salzgehalts, Bestrahlung mit Wellen oder Erwärmung so eingestellt wird, dass die Bindungsaffinität des Adsorbers zu den gebundenen Substanzen zumindest zwischenzeitlich erniedrigt wird und die zu entfernenden Substanzen in Lösung gehen.

15. Verfahren zur Entfernung von unerwünschten Substanzen aus einer biologischen Flüssigkeit umfassend das Dialysieren einer biologischen Flüssigkeit gegen eine Dialysierflüssigkeit durch eine semipermeable Membran, **dadurch gekennzeichnet, dass** die biologische Flüssigkeit durch Zugabe von Säure, Base oder dialysierbarer Substanzen, durch Verdünnung, Veränderung des Salzgehalts, Bestrahlung mit Wellen oder Erwärmung so eingestellt wird, dass die Bindungsaffinität des Trägerproteins zu den gebundenen, zu entfernenden Substanzen erniedrigt wird und die zu entfernenden Substanzen in Lösung gehen.

16. Verfahren gemäß Anspruch 14 oder 15, **dadurch gekennzeichnet, dass** eine Vorrichtung gemäß einem der Ansprüche 1 bis 13 verwendet wird.

17. Verfahren gemäß einem der Ansprüche 14 bis 16, **dadurch gekennzeichnet, dass** als biologische Flüssigkeit Blut oder Blutplasma verwendet wird.

18. Verfahren gemäß einem der Ansprüche 14,16 oder 17, **dadurch gekennzeichnet, dass** als Adsorber humanes Serumalbumin eingesetzt wird.

19. Verfahren gemäß Anspruch 18, **dadurch gekennzeichnet, dass** die Dialysierflüssigkeit humanes Serumalbumin in einer Konzentration von 1 bis 25 g pro 100 ml, bevorzugt 2 bis 10 g pro 100 ml, besonders bevorzugt 4 bis 6 g pro 100 ml, enthält.

20. Verfahren gemäß einem der Ansprüche 14 bis 19, **dadurch gekennzeichnet, dass** als Säure wässrige Salzsäure und als Base wässrige Natronlauge verwendet wird.

21. Verfahren gemäß einem der Ansprüche 14 bis 20, **dadurch gekennzeichnet, dass** der Dialysierflüssigkeit und/oder der biologischen Flüssigkeit dialysierbare, an die zu entfernenden Substanzen bindende Verbindungen zugefügt werden.

22. Verfahren gemäß Anspruch 21, **dadurch gekennzeichnet, dass** zu diesen Verbindungen Coffein und Chelatbildner für Metallkationen gehören.

23. Verfahren gemäß Anspruch 22, **dadurch gekennzeichnet, dass** als Chelatbildner Penicillamin, Trientine, Deferoxamin, Preferiprone, HBED, Vitamin C, BAL, DMPS oder DMSA verwendet wird.
